# NOUVEAU FASCICULE DE BREVET EUROPEEN

(11) **EP 1 131 080 B2**
(45) Date de publication et mention de la décision concernant l'opposition: **15.06.2011**
(45) Mention de la délivrance du brevet: 10.09.2003
(21) Numéro de dépôt: 99956072.5
(22) Date de dépôt: 18.11.1999
(51) Int. Cl.: A61K 35/74, A23C 9/123, C12Q 1/02, A61P 29/00, A61P 1/00

(54) **SELECTION ET UTILISATIONS DE SOUCHES DE BACTERIES LACTIQUES MODULATRICES DE L'IMMUNITE NON-SPECIFIQUE**
SELEKTION UND VERWENDUNG VON MILCHSÄUREBAKTERIENSTÄMMEN, DIE DIE NICHT-SPEZIFISCHE IMMUNITÄT MODULIEREN KÖNNEN
SELECTION AND USES OF LACTIC ACID BACTERIA STRAINS MODULATING NON-SPECIFIC IMMUNITY

(30) Priorité: 18.11.1998 FR 9814471
(43) Date de publication de la demande: 12.09.2001
(73) Titulaire: COMPAGNIE GERVAIS DANONE, 75009 Paris (FR)
(72) Inventeur: CAYUELA, Chantal, F-75016 Paris (FR); DUGAS, Nathalie, F-91370 Verrières-Le-Buisson (FR); POSTAIRE, Eric, F-92170 Vanves (FR)
(74) Mandataire: Vialle-Presles, Marie José
(86) Numéro de dépôt international: PCT/FR1999/002826
(87) Numéro de publication internationale: WO 2000/028943

(56) Documents cités:
- EP-A- 0 965 347
- WO-A-00/23471
- WO-A-96/20607
- WO-A-98/27991
- T. WITTHÖFT ET AL.: "ENTEROINVASIVE BACTERIA DIRECTLY ACTIVATE EXPRESSION OF iNOS AND NO PRODUCTION IN HUMAN COLON EPITHELIAL CELLS." AMERICAN JOURNAL OF PHYSIOLOGY: GASTROINTESTINAL AND LIVER PHYSIOLOGY, vol. 38, no. 3, septembre 1998 (1998-09), pages G564-G571, XP002115076 WASHINGTON, DC, US cité dans la demande
- E. POSTAIRE ET AL.: "ROLE OF NITRIC OXIDE IN THE MUCOSAL IMMUNO-REGULATORY PROPERTIES OF PROBIOTIC STRAINS." FASEB JOURNAL FOR EXPERIMENTAL BIOLOGY, vol. 13, no. 4, 12 mars 1999 (1999-03-12), page A291 XP002137007 BETHESDA, MD US

## Description

L'invention est relative à l'utilisation de bactéries lactiques en tant que régulateurs de l'inflammation de la muqueuse intestinale.

Les bactéries lactiques sont traditionnellement utilisées pour fabriquer des produits alimentaires fermentés, en particulier des produits laitiers.

Il a été rapporté qu'outre leurs qualités nutritionnelles, certains de ces produits alimentaires exerçaient des effets bénéfiques sur la santé ; ces propriétés ont fait l'objet d'un intérêt particulier depuis quelques décennies, et de nombreuses recherches ont été effectuées dans le but de les confirmer, et de définir plus précisément le rôle joué par les ferments lactiques.

Il a ainsi été montré que certaines bactéries lactiques, notamment parmi les lactobacilles et des bifidobactéries, amélioraient l'immunité vis-à-vis d'agents infectieux [PAUBERT-BRAQUET et al., Int. J. Immunother. 11, 153-161 (1995) ; KAILA et al., Dis. Child. 72, 51-53 (1995) ; HUDAULT et al. Appl. Environ. Microbiol. 63, 513-518 (1997)], et possédaient également une activité antitumorale [HAYATSU et al. Cancer Lett. 73, 173-179(1993) ; HOSONO et al. Agric. Biol. Chem. 54, 1639-1643 (1990) ; HOSODA et al. J. Dairy Sci. 75, 976-981 (1992)].

Ces effets ont été notamment attribués à une action sur la composition de la microflore intestinale, au détriment des microorganismes pathogènes, et/ou à une action plus directe sur le système immunitaire, se manifestant en particulier par une augmentation du taux de cytokines activatrices du système immunitaire telles que l'IFNγ ou des interleukines, ainsi qu'une augmentation du nombre de cellules activées intervenant dans la réponse immune spécifique ou non-spécifique, telles que les lymphocytes et les macrophages, et une sécrétion accrue d'immunoglobulines [PERDIGON et al. Int. J. Immunother. 9, 29-52, (1993) ; PORTIER et al., Int. J. Immunother. 9, 217-224 (1993) ; SOLIS PEREYRA et LEMONNIER, Nutr. Research 13, 1127-1140 (1993)] ; DE SIMONE et al., Int. J. Immunother. 9, 23-28 20 (1993) ; PERDIGON et al. J. Dairy Res. 61, 553-562 (1994) ; SCHIFFRIN et al. J. Dairy Sci. 78, 491-497 (1995)].

Cependant, il apparaît que les effets bénéfiques induits par les bactéries lactiques peuvent varier selon l'origine de la pathologie concernée, l'espèce et/ou la souche bactérienne utilisée, et les conditions d'administration. Pour mieux adapter l'utilisation de ces bactéries, ou des produits les contenant, dans le cadre du traitement ou de la prévention de pathologies spécifiques, et pour être en mesure de sélectionner les bactéries les plus appropriées à l'utilisation souhaitée, il est donc nécessaire de mieux connaître les mécanismes par lesquels s'exercent leurs effets.

Les Inventeurs ont entrepris d'étudier l'effet sur la muqueuse intestinale de bactéries lactiques du groupe *Lactobacillus casei* ; ils ont choisi dans ce but la souche de *L*. *casei* DN 114001. Cette souche est décrite dans la Demande PCT WO 96/20607 au nom de : COMPAGNIE GERVAIS DANONE, et a été déposée le 30 décembre 1994, auprès de la CNCM (Collection Nationale de Cultures de Microorganismes) tenue par l'Institut Pasteur, 25 rue du Docteur Roux, à Paris, sous le numéro I-1518, et ses propriétés bénéfiques dans le cadre du traitement des diarrhées ont été montrées.

Les inventeurs ont étudié l'effet *in vitro* de cette souche de *L*. *casei* sur la production de médiateurs de l'immunité non-spécifique (cytokines - pro-inflammatoires et oxyde nitrique) par des entérocytes en culture.

Ces lignées de cellules, qui sont issues de l'épithélium intestinal humain, constituent un modèle d'étude de la réponse de celui-ci à une agression, infectieuse ou autre. Cette réponse se manifeste notamment par la production de cytokines pro-inflammatoires (principalement IL-1, IL-6, TNF-α), et d'oxyde nitrique (NO), généré par une isoforme inductible de la NO synthase (iNOS). L'oxyde nitrique participe, par ses propriétés antimicrobiennes, à la défense contre les microorganismes pathogènes, et lorsqu'il est produit en faible quantité, à la protection de la muqueuse intestinale. Cependant, à forte dose, il diminue la viabilité des cellules épithéliales, et contribue à l'instauration et à l'entretien d'un état inflammatoire chronique [ALICAN et KUBES, Am. J. Physiol. 270, G225-237, (1996) ; TEPPERMAN et al., J. Pharmacol. Exp. Ther., 271, 1477-1482, (1994)]. La production de NO par les entérocytes en culture peut être induite par les cytokines pro-inflammatoires [VALETTE et al., Br. J. Pharmacol., 121, 187-182 (1997) ; KOLIOS et al., Br. J. Pharmacol., 116, 2866-2872 (1995) , ainsi que par les toxines lipopolysaccharidiques (LPS) de certaines bactéries à gram négatif (TEPPERMAN et al, 1994, publication précitée). Des travaux récents [SALZMAN et al. Gastroenterology, 114, 93-102, (1998) ; WITTHOFT et al., Am. J. Physiol., 275, G564-571, (1998)] indiquent que les bactéries entéropathogènes *Escherichia coli, Salmonella dublin, Shigella flexneri,* induisent l'expression de l'iNOS et la production de NO dans des cultures d'entérocytes, pré-activées ou non par des cytokines pro-inflammatoires.

Les inventeurs ont maintenant constaté que, dans le cas de leurs expérimentations avec *L*. *casei*, l'action sur la production de cytokines pro-inflammatoires et de NO variait selon l'état d'activation des entérocytes. En effet, lorsque les cellules sont dans leur état basal, on n'observe aucun effet de *L*. *casei* ; lorsqu'elles sont activées par l'addition de cytokines pro-inflammatoires (ce qui reproduit les conditions d'une agression, infectieuse ou autre), on observe une faible production de NO et de TNF ; cette réponse à l'agression est très significativement augmentée par l'addition de L. *casei*. Enfin, dans le cas de cellules hyperactivées par l'addition de cytokines inflammatoires et de LPS (ce qui reproduit les conditions d'un état inflammatoire pathogène), on observe au contraire une diminution de la production de NO et de TNF, qui est ramenée à un niveau optimal.

Il apparaît donc que cette souche de L. *casei* favorise une réponse adaptative des cellules à une agression, par l'intermédiaire de la modulation de facteurs intervenant dans l'immunité non-spécifique.

La mise en évidence de ces propriétés permet de proposer l'utilisation de la souche CNCM I-1518 de *L*. *casei*, pour l'obtention de compositions inhibitrices d'une réponse inflammatoire pathogène, pour la prévention ou le traitement de pathologies inflammatoires chroniques de l'intestin (colites, entérites, maladie de Crohn, rectocolites hémorragiques, etc.), que ces pathologies soient d'origine infectieuse (induites par des bactéries, des virus, des levures, etc.) ou non ; elles sont particulièrement appropriées dans le cadre du traitement des états inflammatoires chroniques.

Avantageusement, on utilisera une souche capable en outre d'augmenter la production de NO par des cultures d'entérocytes préactivés par des cytokines pro-inflammatoires.

Conformément à l'invention, les bactéries lactiques peuvent être utilisées sous forme de bactéries entières vivantes ou non, sous forme de lysat bactérien, ou sous forme de fractions bactériennes ; les fractions bactériennes convenant à cette utilisation peuvent être choisies en testant leurs propriétés d'augmentation de la production de NO par des cultures d'entérocytes préactivés par des cytokines pro-inflammatoires, et de diminution de la production de NO par des cultures d'entérocytes préactivés par des cytokines pro-inflammatoires et des LPS bactériens.

Préférentiellement, ces compositions peuvent être administrées sous forme de compléments alimentaires. Il peut s'agir notamment de produits laitiers fermentés ; dans ce cas, les bactéries lactiques utilisées, conformément à l'invention, pour l'obtention de ces compositions peuvent faire partie du ferment mis en oeuvre pour l'obtention de ces produits laitiers.

On utilise la souche CNCM I-1518.

De nouvelles souches de bactéries lactiques dotées de propriétés modulatrices de l'immunité non-spécifique, et utilisables notamment pour l'obtention de compositions régulatrices de la réponse inflammatoire des entérocytes, peuvent être obtenues par la mise en oeuvre d'un procédé de criblage comprenant la sélection de souches de bactéries lactiques capables de diminuer la production de NO par des cultures d'entérocytes préactivés par des cytokines pro-inflammatoires et des LPS bactériens.

Avantageusement, ledit procédé comprend en outre une étape de sélection des souches capables d'augmenter la production de NO par des cultures d'entérocytes préactivés par des cytokines pro-inflammatoires, et optionnellement, une étape de sélection des souches n'exerçant aucun effet sur la production de NO par des entérocytes non-activés.

Selon un mode de mise en oeuvre préféré du procédé conforme à l'invention, lesdites souches sont criblées à partir de cultures de bactéries lactiques choisies dans le groupe constitué par les lactobacilles, les lactocoques, les streptocoques et les bifidobactéries.

L'invention englobe également les aliments et compléments nutritionnels, notamment les produits laitiers fermentés contenant ces nouvelles souches, ou des produits dérivés de celles-ci, notamment par lyse et/ou fractionnement cellulaire.

La présente invention sera mieux comprise à l'aide du complément de description qui va suivre, qui se réfère à des exemples non-limitatifs illustrant la mise en évidence des propriétés de la souche CNCM I-1518.

### PROTOCOLES EXPERIMENTAUX GENERAUX :

Les cytokines recombinantes humaines (IL-1β, TNF-α, IFN-γ, 10⁷ U/mg) utilisées proviennent de la société IMMUGENEX (Los Angeles, CA) ; le PDTC (inhibiteur de la formation du facteur de transcription NFκ-B), le L-NAME (inhibiteur des NO-synthases), et le lipopolysaccharide *d'Escherichia coli* proviennent de la société SIGMA (St Louis, MO).

Les kits de dosage par ELISA des cytokines IL-1β, et TNF-α proviennent de la société BIOSOURCE.

Les extraits totaux de *L*. *casei* utilisés lors des expériences sont obtenus par sonication pendant 10 minutes de suspensions de la souche CNCM I-1518, afin de casser les bactéries.

### Culture et stimulation des entérocytes :

Les 2 lignées de cellules de carcinome du colon HT29 et Caco-2 ont été utilisées.

La lignée HT29, initialement isolée par FOGH et TREMPE (Human Tumor Cells *In Vitro,* 115-156, J. FOGH Ed, Plenum Press, New York, 1975) est disponible auprès de la collection ATCC, (Rockville USA), sous le numéro ATCC HTB-38.

La lignée Caco-2, initialement isolée par FOGH (J. Natl. Cancer Inst. 58, 209-214, 1977) est disponible auprès de la collection ATCC, (Rockville USA), sous le numéro ATCC HTB-37.

Légendes des figures :
La figure 1 représente la production de NO par les cellules Caco-2, préactivées (●) ou non (○) par le CYTOMIX, ou par les cellules HT-29 préactivées (■) ou non (□) par le CYTOMIX, en présence de quantités croissantes d'extrait total de la souche CNCM I-1518.
La figure 2 représente l'effet du L-NAME sur la production de NO par les cellules Caco-2 ou par les cellules HT-29 préactivées par le CYTOMIX, en présence ou non d'extrait total (3% v/v) de la souche CNCM I-1518.
( 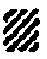 ) + L-NAME
(■) Contrôle
La figure 3 représente l'effet du L-NAME et du PDTC sur la production de TNF par les cellules Caco-2 ou par les cellules HT-29 préactivées par le CYTOMIX, en présence ou non d'extrait total (3% v/v) de la souche CNCM I-1518.
( 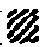 ) + L-NAME
(■) Contrôle
( 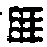 ) + PDTC
La figure 4 représente la production de NO par les cellules Caco-2, préactivées par le CYTOMIX seul (○) ou par le CYTOMIX+LPS (●), ou par les cellules HT-29 préactivées par le CYTOMIX seul (□) ou par le CYTOMIX+LPS (■), en présence de quantités croissantes d'extrait total de la souche CNCM I-1518.

### EXEMPLE 1 : EFFET DE L. CASEI SUR LA PRODUCTION D'OXYDE NITRIQUE PAR LES LIGNEES DE CELLULES EPITHELIALES DU COLON.

Chacune des 2 lignées a été ensemencée à 2x10⁵ cellules/puits en plaques de 96 puits, en milieu DMEM supplémenté de 5% de SVF, de 100 U/ml de pénicilline, de 100 µg/ml de streptomycine et de 2 mM de L-glutamine.

Les cellules sont pré-incubées pendant 24 heures à 37°C, 5% de CO₂, en présence de CYTOMIX (mélange IL-1β : 10 ng/ml, TNF-α : 25 ng/ml, IFN-γ : 10³ U/ml). Les cellules sont ensuite incubées pendant 24 heures supplémentaires en présence ou non de quantités croissantes d'extraits totaux de *L*. *casei* (en % vol/vol).

Après l'incubation, les surnageants de culture sont récupérés et congelés avant la détermination de la concentration en nitrites. Pour certaines expériences, du L-NAME (1 mM), qui est un analogue de la L-arginine et constitue un inhibiteur compétitif spécifique des NO-synthases est ajouté en même temps que les extraits de *L. casei*.

La quantité de NO produit est évaluée par le dosage dans les surnageants de culture, des dérivés stables de ce radical après sa réaction en milieu aqueux: les nitrites et les nitrates. Les nitrates sont dans un premier temps réduits en nitrites par des bactéries exprimant la nitrate-réductase et les nitrites sont ensuite dosés par la méthode de GRIESS. A 100 µl de surnageant on ajoute 100 µl d'une solution composée de 1 volume d'une solution de sulfanilamide à 1% dans l'acide acétique à 30%, et de 1 volume d'une solution de dichlorhydrate de N-1-naphtyl éthylènediamine à 0,1% dans l'acide acétique à 60%. Une courbe de calibration standard est réalisée en présence de différentes concentrations de nitrite de sodium dilué dans du milieu de culture (DMEM 5%SVF). Les absorbances sont ensuite déterminées à 540 nm en utilisant un lecteur MULTISCAN MCC34O, (LABSYSTEM)

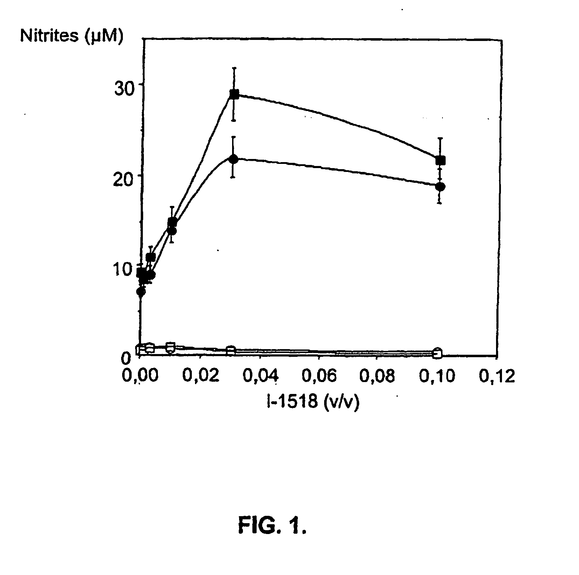

La figure 1 montre qu'en présence du CYTOMIX seul, on n'observe qu'une production limitée de NO par les lignées HT-29 et Caco-2 ; cette production est augmentée de façon dose-dépendante par l'addition de l'extrait de *L. casei*. Un effet maximal est observé pour une concentration d'environ 3% (v/v) d'extrait de *L. casei*. En l'absence de CYTOMIX, *L. casei* n'a pas d'effet sur la production de NO par l'une ou l'autre des lignées.

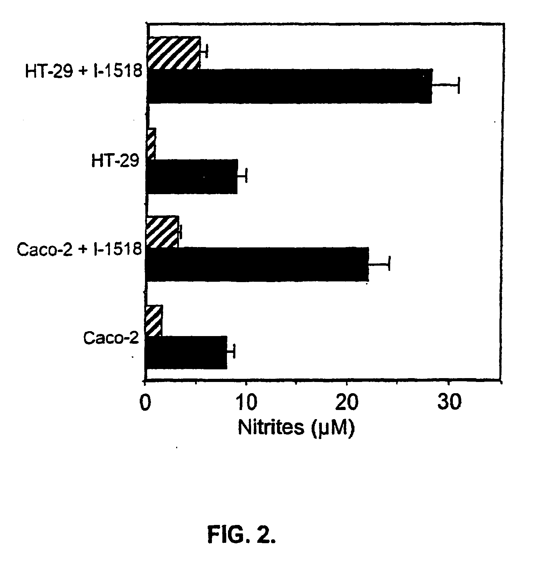

La figure 2 montre que cette production induite par le CYTOMIX est inhibée par l'addition de L-NAME, en présence ou non d'extrait total de *L*. *casei (3%* v/v).

### EXEMPLE 2 : EFFET DE L. CASEI SUR LA PRODUCTION DE TNF-α PAR LES LIGNEES DE CELLULES EPITHELIALES DU COLON.

Chacune des 2 lignées a été ensemencée à 2x10⁶ cellules/puits en plaques de 24 puits, en milieu DMEM supplémenté de 5% de SVF, de 100 U/ml de pénicilline, de 100 µg/ml de streptomycine et de 2 mM de L-glutamine. Les cellules sont ensuite incubées pendant 24 heures en présence de CYTOMIX, puis pendant 24 heures supplémentaires en présence des extraits totaux de *L*. *casei*. Pour certaines expériences, du L-NAME (1 mM) ou un inhibiteur de la voie de transduction NFκB (le PDTC: 10 pM) sont ajoutés en même temps que les extraits bactériens.

Les surnageants de culture sont ensuite récupérés et leur concentration en cytokines déterminées par ELISA.

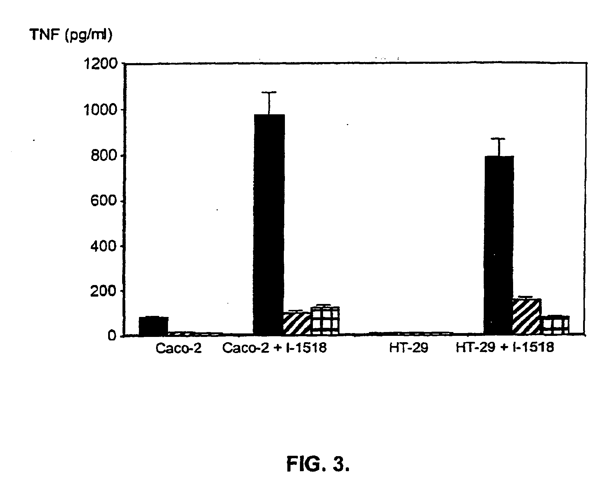

La figure 3 montre qu'en présence du CYTOMIX seul, il n'y a qu'une faible production de TNF-α par la lignée Caco-2, et une absence de production de cette cytokine par la lignée HT-29. Cette production est fortement augmentée, pour les deux lignées, par l'addition d'extrait total de *L*. *casei* ; elle est inhibée par l'addition de L-NAME ou de PDTC, ce qui montre que l'activation de la production de cytokines pro-inflamniatoires par *L. casei* fait intervenir la production de NO et l'activation de NFκB.

Les résultats présentés dans le tableau I ci-après montrent que l'addition de *L. casei* aux cellules préactivées par le CYTOMIX active également la production d'IL-1β.

**TABLEAU I**

| Cellule | Préactivation | Stimulation | IL1-β (pg/ml) | TNF-α (pg/ml) |
|---|---|---|---|---|
| Caco-2 | Aucune | Aucune | ND | ND |
| Caco-2 | CYTOMIX | Aucune | 150±15 | 75±11 |
| Caco-2 | Aucune | CNCM I-1518 | 95±8 | ND |
| Caco-2 | CYTOMIX | CNCM I-1518 | 1254±55 | 975±85 |
| HT-29 | Aucune | Aucune | ND | ND |
| HT-29 | CYTOMIX | Aucune | ND | ND |
| HT-29 | Aucune | CNCM I-1518 | ND | ND |
| HT-29 | CYTOMIX | CNCM I-1518 | 908±63 | 789±45 |
| ND : NON DETERMINE | | | | |

### EXEMPLE 3 : EFFET DE L. CASEI EN PRESENCE DE LPS DE BACTERIES GRAM⁻, SUR LA PRODUCTION D'OXYDE NITRIQUE PAR

### LES LIGNEES DE CELLULES EPITHELIALES DU COLON PRE-ACTIVEES PAR DES CYTOKINES PROINFLAMATOIRES.

Le protocole est identique à celui de l'exemple 1 ci-dessus, à ceci près que 10 µg/ml de LPS d'*E*. *coli* sont ajoutés lors de l'incubation avec l'extrait total de *L. casei*.

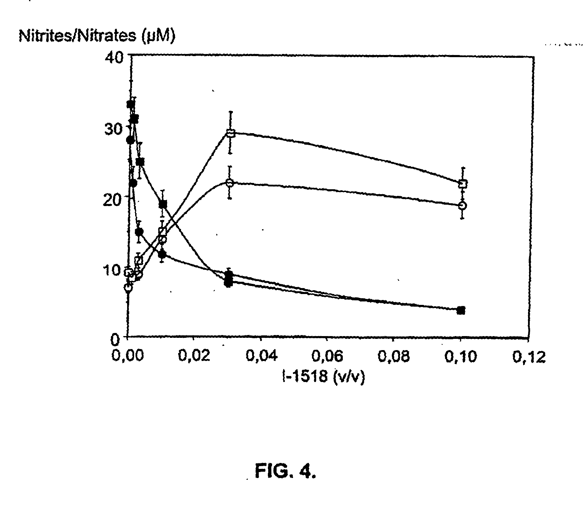

Les résultats sont illustrés par la figure 4, qui montre une production importante de NO en l'absence de *L. casei* (cellules stimulées par CYTOMIX + LPS), qui diminue en présence de quantités croissantes de *L*. *casei*, jusqu'à revenir au niveau de celle des cellules activées par les cytokines seules.

## Revendications

1. Utilisation de bactéries lactiques de la souche *L*. *casei* CNCM I-1518, pour l'obtention d'une composition pour prévenir ou traiter des pathologies inflammatoires chroniques de l'intestin.

2. Utilisation selon la revendication 1, **caractérisée en ce que** ladite composition est sous la forme d'un complément alimentaire.

3. Utilisation selon l'une quelconque des revendications 1 à 2, **caractérisée en ce que** ladite composition est sous la forme d'un produit laitier fermenté.

4. Procédé de criblage de nouvelles souches de bactéries lactiques dotées de propriétés modulatrices de l'immunité non-spécifique, **caractérisé en ce qu'**il comprend la sélection de souches de bactéries lactiques capables d'inhiber la production de NO par des cultures d'entérocytes préactivés par des cytokines pro-inflammatoires et des LPS bactériens.

5. Procédé selon la revendication 4, **caractérisé en ce qu'**il comprend en outre une étape de sélection des souches capables d'augmenter la production de NO par des cultures d'entérocytes préactivés par des cytokines pro-inflammatoires, et optionnellement, une étape de sélection des souches n'exerçant aucun effet sur la production de NO par des entérocytes non-activés.

6. Procédé selon l'une quelconque des revendications 4 ou 5, **caractérisé en ce que** lesdites souches sont criblées à partir de cultures de bactéries lactiques choisies dans le groupe constitué par les lactobacilles, les lactocoques, les streptocoques et les bifidobactéries.

## Claims

1. Use of lactic bacteria of the strain *L*. *casei* CNCM 1-1518 in order to obtain a composition for preventing or treating chronic inflammatory pathologies of the intestine.

2. Use according to claim 1, **characterised in that** the said composition is in the form of a food supplement.

3. Use according to either of claims 1 to 2, **characterised in that** the said composition is in the form of a fermented dairy product.

4. Method for screening new strains of lactic bacteria possessing non-specific immunity-modulating properties, **characterised in that** it comprises the selection of strains of lactic bacteria capable of inhibiting the production of NO by culture of enterocytes pre-activated by pro-inflammatory cytokines and bacterial LPSs.

5. Method according to claim 4, **characterised in that** it additionally includes a step for selecting strains capable of increasing the production of NO by cultures of enterocytes pre-activated by pro-inflammatory cytokines and, optionally, a step for selecting strains exerting no effect on the production of NO by non-activated enterocytes.

6. Method according to either of claims 4 or 5, **characterised in that** the said strains are screened from cultures of lactic bacteria chosen from the group consisting of lactobacilli, lactococci, streptococci and bifidobacteria.

## Patentansprüche

1. Verwendung von Milchsäurebakterien des *L. casei*-Stammes CNCM I-1518 zur Herstellung einer Zusammensetzung zur Vorbeugung oder Behandlung von chronischen inflammatorischen Darmpathologien.

2. Verwendung nach Anspruch 1, **dadurch gekennzeichnet, dass** die Zusammensetzung in Form eines Nahrungsergänzungsmittels vorliegt.

3. Verwendung nach einem der Ansprüche 1 oder 2, **dadurch gekennzeichnet, dass** die Zusammensetzung in Form eines fermentierten Milchprodukts vorliegt.

4. Screeningverfahren auf neue Milchsäurenbakterienstämme, die Eigenschaften zur Modulation unspezifischer Immunität besitzen, **dadurch gekennzeichnet, dass** es die Selektion von Milchsäurebakterienstämmen umfasst, die befähigt sind, die NO-Produktion von Enterozytenkulturen, die durch proinflammatorische Zytokine und bakterielle LPS präaktiviert sind, zu inhibieren.

5. Verfahren nach Anspruch 4, **dadurch gekennzeichnet, dass** es außerdem einen Selektionsschritt auf Stämme umfasst, die befähigt sind, die NO-Produktion von Enterozytenkulturen, die durch proinflammatorische Zytokine präaktiviert sind, zu erhöhen, und gegebenenfalls einen Selektionsschritt auf Stämme, die keinen Effekt auf die NO-Produktion von nichtaktivierten Enterozyten ausüben.

6. Verfahren nach irgendeinem der Ansprüche 4 oder 5, **dadurch gekennzeichnet, dass** die Stämme ausgehend von Milchsäurebakterienkulturen gescreent werden, die ausgewählt sind aus der Gruppe, die aus Laktobazillen, Laktokokken, Streptokokken und Bifidobakterien besteht.
